# EUROPEAN PATENT APPLICATION

(11) **EP 4 356 879 A1**
(43) Date of publication of application: **24.04.2024**
(21) Application number: 22201815.2
(22) Date of filing: 17.10.2022
(51) Int. Cl.: A61F 5/01

(54) **ANATOMIC-PHYSIOLOGICAL SEPARATION OF TOES**

(71) Applicant: Frontela Carreras, Luis, 41009 Seville (ES)
(72) Inventor: Frontela Carreras, Luis, 41009 Seville (ES)
(74) Representative: Bartrina Diaz, José María

(57) **Abstract**

Anatomic -physiological separators of toes and the procedure to obtain them. Considered as medical surgical equipment adapted to the requirement of the population in all their varieties. Their use is aimed at cushioning discomfort and deformities caused by pathologies, defects or amputations of toes. They are adapted to the shape and size of the interdigital surfaces and reproduce the interdigital surfaces on which they are based. A series of previously made models or prototypes are then massed produced. They represent each and every one of the interdigital spaces of both feet and of all types of feet, as a result of combining the size or shape of the feet, sex, age, their digital formula: square, Greek, Egyptian and ideal foot, as well as the metatarsal formula: Minus index, minus plus index, plus index.

## Description

### OBJECT OF THE INVENTION

The following invention as expressed in the title of this descriptive memory, is aimed at providing an element which allows the separation of one toe from another/others, fulfilling a series of anatomic-physiological peculiarities, which can also be used as ways of treatment and prophylaxis, to alleviate deformities or suffering caused by pathologies, defects or amputations of toes.

The field of application of this invention, is in surgical medical equipment, particularly conceived for the protection of pathologies, affections or existing irregularities in toes.

### BACKGROUND TO THE INVENTION

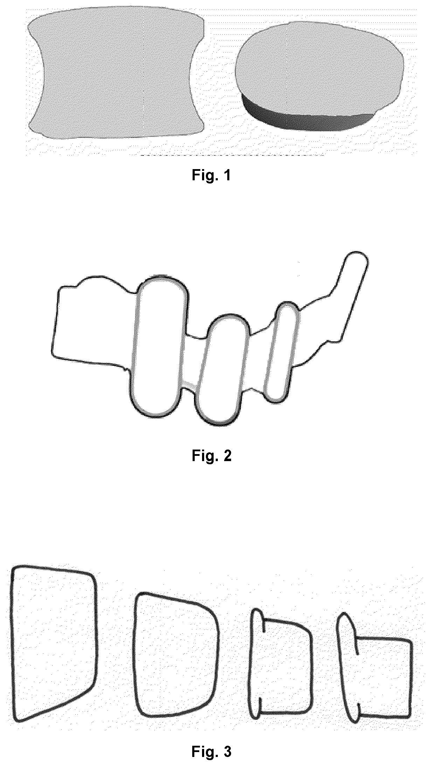

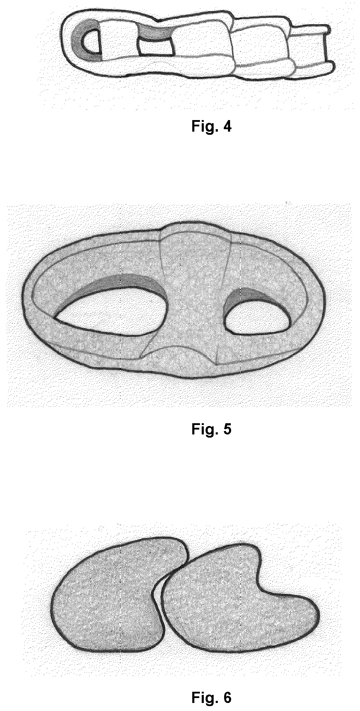

As regards the background to the state of the art, the methods or elements which have so far been used as toe separators, and are used to avoid or cushion the contact or the pressure which cause discomfort, pain or impede the curing of injuries or affections, these do not comply with the anatomic or physiological requirements to be used efficiently. These can be classified, among others, in the following types:
a) Cylindroid or hyperboloid devices.
   They essentially have a cylindrical form with lateral concave walls as is shown in Figure 1.
b) In form of wedges for each interdigital space.
   Isolated or, very frequently, joined together by a band going round the perimeter of the foot at the level of the proximal phalanges or the metacarpophalangeal joints, as shown in Figures 2-3.
c) Cylindrical
   Isolated, or joined together at the four interdigital spaces corresponding to each foot as shown in Figure 4, some with two toes joined as is shown in Figure 5.
d) Flat multiform ones.
   Flat separators as are shown in Figure 6.
e) Some other diverse ones
   With simple varied forms and even "household" remedies.

Regarding the present inventions in the state of the art, the following documents are included and which are identified by number of publication and title, respectively:
- ES-1035012_U, "Separator device and protector for toes".
- ES-1287604_U, "ORTHOPEDIC DEVICE FOR BUNIONS".
- ES-2774372_T3, "Device for holding and/or protecting fingers or toes".

If we delve into some of the unresolved problems from the present state of the art and as an example, we can see a left foot with the second toe amputated. A toe separator was used for several years as shown in Figure 7. The first toe shows a deviation of 29.3°, which, compared to the position of the same toe on the right foot, which is at 9.8°; this represents a deviation of 19.5°, which was not the sufficiently corrected by placing the corrector as shown in Figure 8. Then, and in conclusion, once the separator was removed, as is shown in Figure 9, the first toe of the left foot happened to show a deviation regarding the same toe of the right foot, of 35.1° - 9.8° = 25.3°, hence the separator which was used did not serve its purpose.

Additionally, the separators present in the state of the art, independently as to whether they adapt to the real anatomy of the interdigital spaces and the different types of feet in which they are going to be used, as has been demonstrated in the previous example; they have failed to take into account the physiology of the movements.

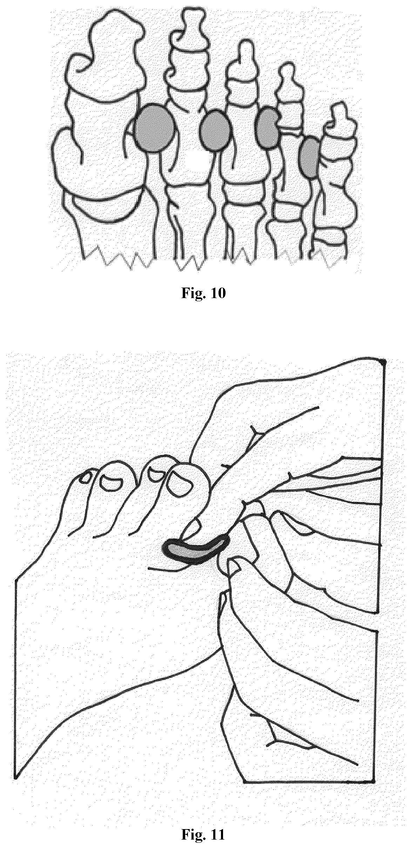

In fact, by using systems which are merely based on the observation of books or anatomical drawings, without taking into account the physiology of the movements associated with the toes which are in contact, also leads to notable errors. For example, in anatomy books, we can observe the formation of some ovals or ellipses in toe skeletons, which might mistakenly lead us to believe that the ideal models of separators would be the ellipsoids shown in Figure 10. This is incorrect, as it ignores the fact that, as well as bones, there are vessels, nerves, muscles, ligaments and other soft structures which have to be protected as much or even more than the bones.

In summary, such is the lack of scientific rigour and such is the simplicity of the current toe separation procedures, that even in Internet, home remedies of separators can be found, which consist of placing pieces of sponge between the toes.

So in conclusion, the toe separator devices which are currently available in the state of the art, are simple objects with diverse shapes and sizes, all of which have flat surfaces, which goes against anatomical reality. You only have to examine the interdigital surfaces, where the toe separators should be placed, to see that these surfaces are far from flat and even, in fact they are convex. If they are examined with a magnifying glass, you can see that there are light concavities and convexities, as well as subtle marks on the skin. Hence, the current toe separators can never be adapted to the surfaces between two toes. There would be empty spaces and others in which the separator would wield greater pressure, which would make it more difficult to obtain the appropriate treatment and pain relief. Those currently in use are employed to separate any toe, although they are of different sizes, and are used for any size of foot and any type of foot, whether the lengths of each and every toe is regular or irregular in relation to the rest. They are the same for all feet, without distinguishing the regularity or irregularity of the metacarpal lengths, in which case they do not take into account the anatomy and physiology of each foot to adapt it to each person's particular need. It is as if everybody was obliged to wear the same size shoe, ignoring the size of each foot, which leads to notorious limitations and does not allow us to fulfil the purpose for which they were designed.

Therefore, from the aforementioned, the "anatomic -physiological toe separators" used as utensils to separate a toe from another/others, fulfil a series of anatomic-physiological peculiarities and, in comparison with the current state of the art, offer a unique and advantageous way of treatment in numerous circumstances, such as:
- To restore the alignment of toes, when one of the toes is displaced from the rest.
- To avoid rubbing, irritation, friction between toes or other irritation abrasions through contact.
- To reduce the discomfort caused by sores, hyperkeratosis and other inflammatory, infectious or pathologic processes of any type which should be protected from any rubbing or contact.
- As a way to keep the interdigital spaces dry
- When the chiropodist indicates that it would be appropriate to use a toe separator, and even when the patient, for diverse circumstances, requests it
- Therapists and others, who with advances in science, recommend its use in certain treatments and prophylaxis.

With this goal, the "Anatomic-physiological separators of toes" base their anatomic-physiological properties on the real anatomy of the interdigital spaces. They take into account the physiology of the movement of these and the different types of feet in which they are going to be used and make models or prototypes, which would later be massed produced, with a sufficient representation of all population groups, by combining the following classifications:
a) Anatomic.
   They reproduce the anatomy of the surfaces on which they are placed, that is to say, those which delimit the interdigital spaces where they are going to be placed
b) Physiological.
   In order to better serve the physiology of the feet, the same model cannot be used for all feet, it is necessary to make a model for each type of foot, classified according to:
   - The size of the feet, referring to the shoe size, covering at least sizes 16 to 47 in Europe (EU) or their equivalent in different countries in the world. These sizes could be increased depending on their evolution with the passing of time.
   - Sex: male, female.
   - The age, to begin with, adults and children. If it is considered necessary, models could be made per each decade of life, and if considered suitable, they could be divided into shorter periods of time.
   - The digital formula of the feet.
      The digital and metatarsal formulas allow us to classify the different types of feet, as regards the variability of the distal end of the toes and of the metatarsals.
      According to the digital formula, the following types of feet are distinguished:
      ✔ Square foot, when the first and second toes are of equal length and the rest are gradually shorter.
      ✔ Greek foot, when the first toe is shorter than the second one and the rest are gradually shorter in relation to the second one.
      ✔ Egyptian foot, when the big toe is longer than the second one and the rest progressively shorter.
         Ideal foot according to the formula of Maestro (Maestro M. Besse J.L. Ragusa M. Berthonnaud E. Forefoot morphotype study and planning method for forefoot osteotomy. Foot Ankle Clin N AM 2003; 695-710*).*
         The second metatarsal is approximately 4 mm longer than the third, and the third 6 mm longer than the fourth, and the fourth 12 mm longer than the fifth.
   - Metatarsal formula.
      There are three types of situations of the distal epiphysis of the metatarsals:
      ✔ *Minus index*, where the first metatarsal is shorter than the second and the rest are shorter and shorter.
      ✔ *Plus minus index*, when the first and second metatarsal are very much the same.
      ✔ *Plus index*, if the first metatarsal is longer than the second.

Any of these types of metatarsal or digital formulas are normal and can be combined.

### EXPLANATION OF THE INVENTION

To explain the invention, it can be said that the "Anatomic-physiological toe separators" provide medical surgical equipment adapted for all the requirements of the population, with the aim of cushioning with their use the deformities and discomfort caused by pathologies, defects or amputations of toes. The size and shape are accordingly adapted to reproduce the interdigital surfaces on which they are placed. It is based on the mass production of a series of models which have previously been identified and produced, representing all and each one of the interdigital spaces of both feet and of all types of feet, as a result of combining the following classifications:
1. The shape or size of the feet, according to sizes 16 to 47 in Europe (EU) or their equivalent in other countries, which could be added as required.
2. The sex.
3. The age, both adults and children, notwithstanding the other classifications by age range.
4. The digital formula of the feet; square,Greek, Egyptian and ideal foot.
5. The metatarsal formula: *Minus index, minus plus index, plus index.*

Obviously, the common characteristics which give rise to different models of interdigital space will be included in the corresponding sub-classifications.

In order to produce the "Anatomic physiologic toe separators", it is necessary to carry out prototypes adapted to all the varieties of requirements of the population, using the following procedure:
1. Collection of data: including the case number, age, sex, foot size, type of foot according to the digital and metatarsal formulas, abnormalities observed and any other data of interest in the investigation.
2. Each one of the interdigital spaces is moistened with oil or any other lubricant so as to be able to easily extract the modelling material or putty used for this purpose.
3. A slice of modelling putty is cut in an appropriate size and shape, beginning with 5, 8, 10 and 15 mm thickness, or any other material which is ideal for modelling and this is placed between each two toes which make up the 1^{st}, 2^{nd}, 3^{rd} and 4^{th} interdigital spaces of each foot. The thickness will be increased in size and modified based on the necessities which arise when carried out in populations from different races and particular situations.
4. The piece of modelling putty is put in place, applying pressure until it reaches the bottom of the interdigital space and the two toes are pressed together, applying greater pressure towards the proximal side of this space.
5. Using an appropriate instrument, the excess putty is removed from the perimeter of the prototype of the interdigital space, leaving a 1 to 3 mm margin, except in the upper part where the surface is left at the same level as the toes.
6. You wait an appropriate length of time, or it can be dried with hot air, and so it can be removed without damaging it. You obtain eight interdigital space models, four for each foot corresponding to the peculiarities of the foot.
7. So that each separator is not displaced, a piece of hook or the rough side of a Velcro tape is stuck to the upper surface. Then the foot is held in place, placing on top of the separator, the fragment which corresponds to the smooth side or loop of the Velcro tape.

As many models as possible are made of each size, feet formulas and different peculiarities, so as to select for production those which have most characteristics in common for each group and type of foot.

Obviously, as part of the process of making the prototypes, each fragment has to be inspected, correcting any imperfection which is not found in the interdigital spaces and discarding those which cannot be recovered.

Additionally, the mass production of each model, may include on the surfaces of the interdigital space, a thin layer of attached cotton with the idea that an antibiotic, anti-inflammatory or any medication or therapeutic substance can be added.

### DESCRIPTION OF FIGURES

To complement the descriptions that have been made and to help to better understand the characterisitics of the invention, which is by all means a practical one, drawings have been included by way of illustration and without limit which represent the following:
Figure 1.-Cylindroid devices.
Figure 2.- Wedge shaped devices for each interdigital space.
Figure 3.- Another wedge shaped device for each interdigital space.
Figure 4.- Cylindrical devices
Figure 5.- Cylindrical devices supporting two toes.
Figure 6.- Configuration device in flat multiforms.

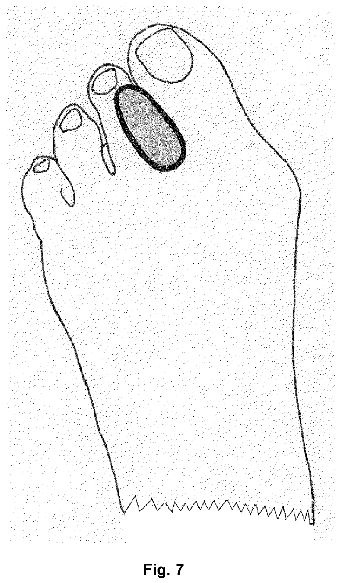

Figure 7.- Foot with second toe amputated, with a cylindrical separator available on the general market attached to the toe for approximately three years.

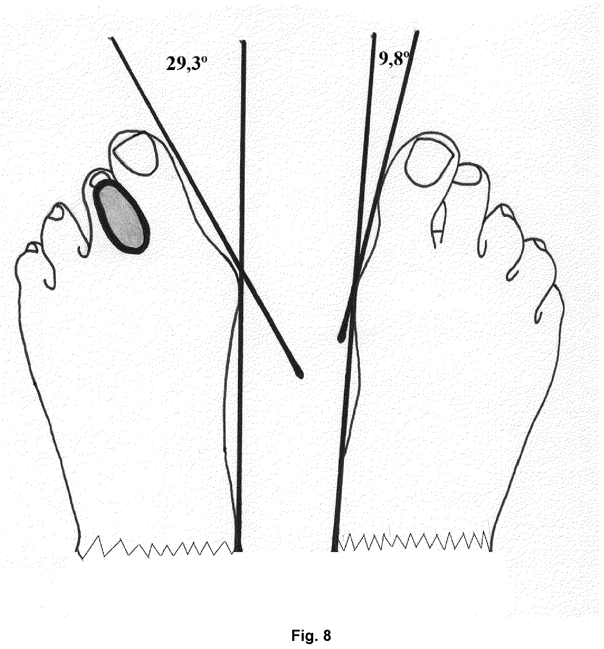

Figure 8.- First toe of the foot, with the 2nd toe amputated, with a cylindrical separator attached to the first left interdigital space and with time deviated 19.5° to the left in relation to the foot with no toe amputated.

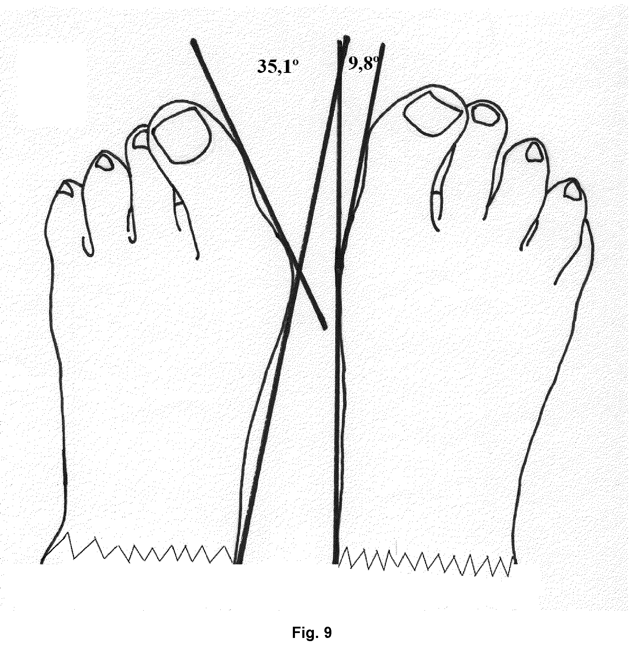

Figure 9.- Shows both feet when the toe separator was taken off, showing how the deviation of the first toe of the left foot increased.
Figure 10.- Shows a main view of a right foot, to demonstrate how the existing ellipsoid toe separators, available on the market, do not adapt to the 3^{rd} and 4^{th} interdigital spaces.
Figure 11.- Show how the oil or similar substance is applied to the interdigital spaces, where the modelling piece will be placed.

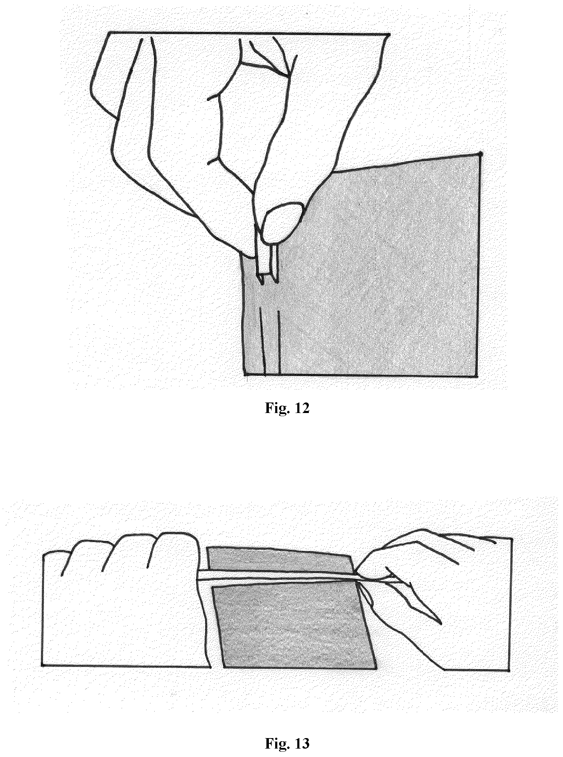

Figure 12.- Shows how you mark the width of the putty to be cut.
Figure 13.- Shows how to cut the piece of modelling putty, to be placed in each interdigital space.

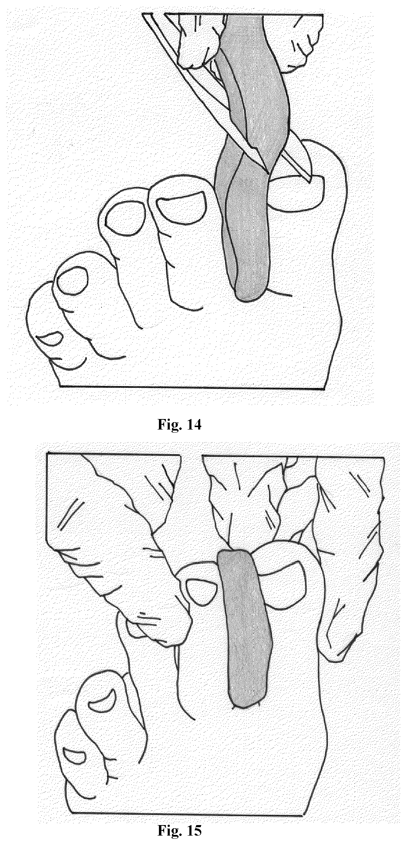

Figure 14.- Shows how you cut the fragment to be placed in the interdigital space.
Figure 15.- Shows how you introduce the putty fragment in the interdigital space and how it is displaced to the most proximal part.

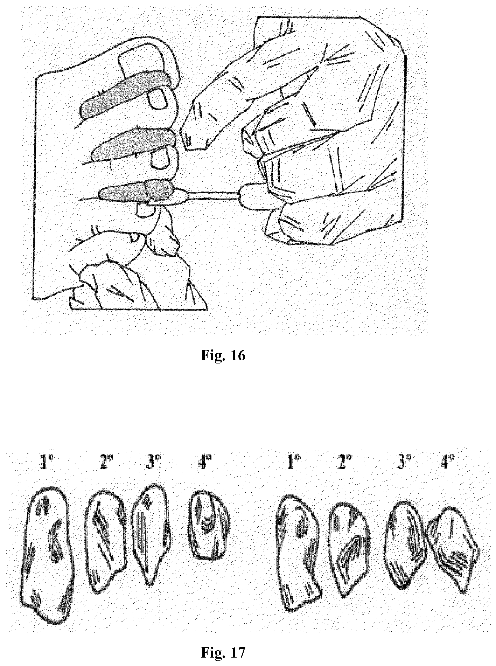

Figure 16.- Shows how you take away the excess putty which does not affect the surfaces of the interdigital spaces, using a suitable tool.
Figure 17.- Shows the resulting prototypes from the eight interdigital spaces of a person's feet, from the right to left foot and left to right foot with eight different separators, for each combination of feet size, sex, age, digital formula of the feet and metatarsal formula.

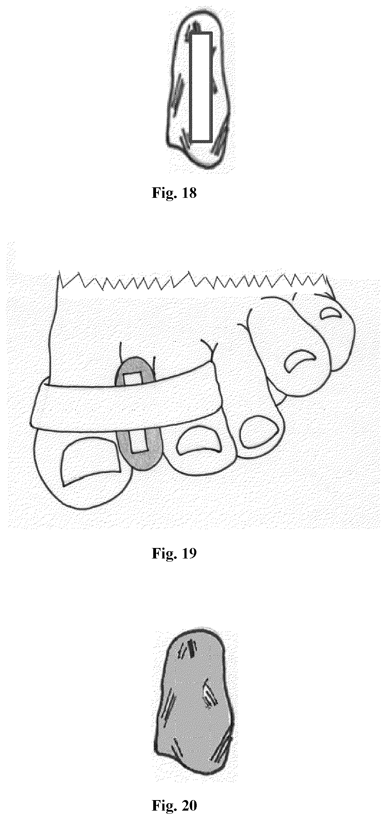

Figure 18.- Shows how you attach to the upper surface of each separator, a hook fragment or the rough side of a Velcro tape.
Figure 19.- Shows how the foot is held in place, placing on top of the separator the fragment which corresponds to the smooth edge of the Velcro tape.
Figure 20.- Shows an anatomic- physiological separator of toes which on the surfaces of the interdigital spaces includes a thin piece of cotton to allow us to add an antibiotic, anti-inflammatory or any other medication or therapeutic substance which might be required.

### EXAMPLE OF PREFERENTIAL USE

As regards the preferential use of "Anatomic-physiological toe separators", in the light of the drawings which we have commented on, this can be done using the following sequence:
A. Making various prototypes adapted to the population
   So as to include the diverse anatomic physiological varieties, we will make as many models as is necessary of each foot size and these will be related to the sex, shoe size or foot size, both in adults and in children, the digital formula of the feet (square foot,
   Greek foot, Egyptian foot, ideal foot), the metatarsal formula (Minus index, plus minus index, plus index), deploying the following sequence:
   PHASE 1-Data Collection
      To begin with, we take the data of each person who is going to undergo an interdigital space model, such as the case number, age, sex, foot size, foot type according to the digital and metatarsal formulas, any anomalies observed and any other data of interest for the investigation.
   PHASE 2-Moisten the skin with oil
      In order to avoid the modelling material sticking to the skin, it is moistened with oil, liquid paraffin or any other similar substance so it will be easy to remove, as is shown in Figure 11. We have to avoid applying too much oil to the skin surface, removing any surplus oil with cotton wool or any other material which does not leave fibres or foreign bodies.
   PHASE 3- An appropriate sized piece is cut for modelling.
      Modelling putty is used, or any other material which is ideal for modelling, such as silicone, alginate, plaster, putty etc. can be used, including clay, wax, plasticine, or modelling paste. Or any other which exists in the market. It is cut into the correct shape according to the length of the interdigital spaces and is placed between each two toes which make up the 1st, 2nd, 3rd and 4th interdigital space of each foot.
      Each interdigital space has to be carefully modelled. Based on the experienced acquired during years of exercising the speciality of traumatology and orthopedics, we believe that, at first, it is useful to make models with four thicknesses of 5, 8, 10 and 15 millimetres. However, in some cases, it is advisable to use other sizes, due to necessities which come up in diverse races and particular situations.
      Once you have marked where the cut is to be made, as is seen in Figure 12, a piece of modelling putty or other material is cut as is seen in Figure 13.
   PHASE 4-A fragment of this slice is taken, of a chosen thickness and usually rectangular or triangular, or of a different morphology, and it must be sufficiently hydrous to be easily malleable, so that it can be closely adapted to the two surfaces of the interdigital space on which it is applied, as is shown in Figure 14.
   PHASE 5-The modelling putty is spread and by applying pressure is pressed to the end of the interdigital space as is shown in Figure 15 and the two toes are pressed together, at first with gentle pressure on the most distal part of the toes and progressively increasing towards the proximal part, to make sure that the very last detail of the two surfaces forming the interdigital space is recorded in the putty. You must take great care not to produce deformations by excess or lack of care.
   PHASE 6-With an appropriate instrument, the excess putty should be removed from the whole perimeter of the prototype of the interdigital space, leaving a border of one to three millimetres, except in the upper part where the surface is left level with the toes to allow the support to be attached, as is shown in Figure 16.
   PHASE 7- The model is left sufficient time for it to dry, so it can be taken out without deforming it, otherwise it can be dried using hot air.
      In this way and with sufficient training and practice, the four models of interdigital spaces are obtained of each foot as is shown in Figure 17 and are then given a quality control test, and those which have some type of defect are discarded.
   PHASE 8- So as to hold the anatomic-physiological separators in the correct place, and to avoid them being displaced, the rough side, or hook, of a Velcro tape is attached to the upper side of each separator, as is shown in Figure 18, which at the same time the smooth side, or loop, of the Velcro tape is attached to the corresponding foot, as is shown in Figure 19.
B. Mass production for each prototype
   Once as many models as possible of each size, of feet formula and of diverse peculiarities have been produced, we have to select for manufacturing those that have the most common characteristics of each group or type of foot.
   Once we have the representative prototypes of the anatomic-physiological varieties present in the population, each prototype will be mass produced using the manufacturing technology in 3D printers with silicone, using moulding techniques or other techniques which allow us to make copies in silicone, or in another material with similar characteristics, with a hardness of between 20 and 40, measured with a durometer Shore C. However, in the case of manufacturing with silicone, the hardness can be above or below these levels according to its planned use in the market.
   Alternatively, the separators used in this invention, will have a thin piece of cotton attached to the surface of the interdigital space, as can be seen in Figure 20 so as to be able to add an antibiotic, anti-inflammatory or any other medication or therapeutic substance as required.
C. At the disposal of the professional doctor
   The "Anatomic-physiological separators of the toes" are purchased by hospitals or specialists as part of their medical surgical equipment. Their aim is the protection of pathologies, affections or irregularities in toes, using a catalogue where all the varieties are classified.
   By way of example, the doctor or chiropodist in the surgery takes the data of those patients who require treatment in the interdigital spaces of their foot. Data such as age, sex, foot size, type of foot according to the digital and metatarsal formulas, as well as the abnormalities observed. From this data and with the help of the catalogue, they can make use of the available separator which best adapts from the anatomic-physiological point of view to the necessities of their patient.

We do not feel that it is necessary to expand this description of the material, as any expert in this field will understand the advantages of this invention in its different applications. Besides, the material used, dimensions, geometries, different designs or elements for holding the foot, type of Velcro, as well as the process of obtaining each prototype or its later mass production, are always susceptible to change as long as this does not involve an essential modification of the invention. The terms in which the description have been written should be understood in a wide and not a limited sense.

## Claims

1. Anatomic-physiological separators of toes, which are conceived to be used as medical surgical equipment, have been adapted to the different requirements of the population, in all their varieties, with the aim of cushioning the deformities and discomfort caused by pathologies, defects or the amputation of toes. The configurations are adapted in shape and size to reproduce their interdigital surfaces and are based on the mass production of a series of models which have previously been made and which represent each and every one of the interdigital spaces of both feet of all types of feet, as a result of combining the following classifications:
A. The size or shape of the feet.
B. Sex.
C. Age, both adults and children, even though other classifications are made for age ranges.
D. The digital formula of the feet: square, Greek, Egyptian and ideal feet.
E. The metatarsal formula: *Minus index, minus plus index, plus index.*

2. Anatomic-physiological separators of toes, according to vindication 1, **characterised by** the making of prototypes adapted to all their varieties and those required by the population, by means of applying the following procedure:
A. Collection of data: including the case number, age, sex, foot size, type of foot according to the digital and metatarsal formulas, the abnormalities observed and any other data of interest for the investigation.
B. Moistening each one of the interdigital spaces with oil or any other lubricant so as to allow us to easily remove the modelling material or putty used for this purpose.
C. Cutting the slice of modelling putty into an appropriate size and shape, starting with a thickness of 5, 8, 10, and 15 mm, or any other material which can be used for modelling and which are placed between every two toes which make up the 1^{st}, 2^{nd}, 3^{rd}, and 4^{th} interdigital spaces of each foot. The thickness can be increased or modified according to the necessities in populations of different races and special situations.
D. Introducing the slice of modelling putty, and applying pressure until it reaches the bottom of the interdigital space and applying an adequate pressure to join the two toes from this space, and this pressure will be greater towards the proximal side of this space.
E. Removing, with a suitable instrument, the surplus putty around the whole perimeter of the prototype of the interdigital space, leaving a margin of 1 to 3 millimetres, except on the top part where the surface is left at the same level as the toes.
F. Removing the moulds in perfect condition, after waiting a suitable length of time, or after drying with hot air. Eight models are obtained of the interdigital spaces, four for each foot, which correspond to the peculiarities of each foot.
G. Each separator is held in place on the upper surface, to prevent it from moving, by means of a hook or the rough side of a Velcro tape and the foot is secured, by placing the smooth side or loop of a Velcro tape on top of the separator.

3. Anatomic-physiological separators of toes, according to vindication 1 and 2, are **characterised by** sticking on the surfaces of the interdigital space, a fine layer of cotton, to enable us to add an antibiotic, anti-inflammatory or any medication or therapeutic substance as may be required.
